# EUROPÄISCHE PATENTSCHRIFT

(11) **EP 2 714 018 B1**
(45) Veröffentlichungstag und Bekanntmachung des Hinweises auf die Patenterteilung: **28.08.2019**
(21) Anmeldenummer: 12723144.7
(22) Anmeldetag: 14.05.2012
(51) Int. Cl.: A61K 9/70, A61K 47/12, A61K 31/485, A61P 25/04, A61P 25/36

(54) **TRANSDERMALES THERAPEUTISCHES SYSTEM ENTHALTEND BUPRENORPHIN UND EINE ALPHA-HYDROXYSÄURE**
TRANSDERMAL THERAPEUTIC SYSTEM FOR ADMINISTERING BUPRENORPHIN COMPRISING A ALPHA-HYDROXY-ACID
SYSTEME TRANSDERMIQUE COMPRENANT DE LA BUPRENORPHINE AINSI QU'UN ALPHA HYDROXY ACIDE

(30) Priorität: 27.05.2011 DE 102011076653
(43) Veröffentlichungstag der Anmeldung: 09.04.2014
(73) Patentinhaber: Luye Pharma AG, 83714 Miesbach (DE)
(72) Erfinder: FAHRMEIR, Julia, 80779 München (DE); SCHURAD, Björn, 81667 München (DE)
(74) Vertreter: Zehetner Patentanwälte
(86) Internationale Anmeldenummer: PCT/EP2012/058950
(87) Internationale Veröffentlichungsnummer: WO 2012/163665

(56) Entgegenhaltungen:
- EP-A1- 1 174 137
- EP-A2- 0 368 409
- WO-A2-2007/011763
- US-A1- 2007 298 091

## Beschreibung

Gegenstand der Erfindung ist ein System zur transdermalen Abgabe von Buprenorphin enthaltend eine α-Hydroxysäure und als weitere polymere Komponente Polyvinylpyrrolidon. Das transdermale therapeutische System (TTS) ermöglicht eine kontinuierliche Abgabe von Buprenorphin in einer therapeutisch wirksamen Dosis über einen Zeitraum von mindestens drei Tagen und zeichnet sich insbesondere durch eine Polymermatrix aus, in welcher der Wirkstoff physikalisch stabilisiert vorliegt.

Die transdermale Gabe von Opioiden hat bekanntermaßen den Vorteil, dass das Medikament nicht über Kanülen in den menschlichen Körper eingebracht werden muss und daher einfach und ohne Schmerzen appliziert werden kann. Gleichzeitig ist es jedoch von Bedeutung, dass der Wirkstoff in ausreichenden Mengen kontinuierlich aus dem System abgegeben wird und die Zufuhr durch das Entfernen des Systems möglichst einfach unterbrochen werden kann.

Um eine kontinuierliche und ausreichende Abgabe des Wirkstoffs aus dem transdermalen System auch über einen Zeitraum über 24 h hinaus zu gewährleisten, muss die Beladung des Systems mit einer Wirkstoffmenge, welche häufig nahe der Sättigungslöslichkeit des Wirkstoffs in der Polymermatrix liegt, erfolgen. Darüber hinaus ist zu beachten, dass die Diffusion des Wirkstoffs durch die menschliche Haut nur in gelöster Form erfolgen kann. Dies erweist sich häufig als problematisch, da aufgrund einer geringeren Wirkstoffbeladung in der Polymermatrix zumeist die Wirkstoffabgabe aus dem System nicht ausreichend ist oder eine Wirkstoffbeladung nahe oder im Bereich der Sättigungslöslichkeit zur Kristallisation des Wirkstoffs führen kann und somit eine vorhersagbare bzw. definierte Wirkstoffabgabe nicht ermöglicht. Dieser Aspekt ist umso wichtiger, als gerade Opioide wie das nur schwer hautgängige Buprenorphin einen sehr geringen therapeutischen Index aufweisen und bei Unterdosierung nicht effektiv wirksam sind bzw. bei Überdosierung aufgrund des Nebenwirkungsprofils zu starker Beeinträchtigung des Patienten führen können.

Des Weiteren können Wirkstoffkristalle, welche sich auf Grund zu hoher Beladung durch Rekristallisation des Wirkstoffs bilden können, die Klebeeigenschaften des TTS wesentlich beeinträchtigen und zu Hautirritationen führen.

Transdermale therapeutische Systeme zur Verabreichung des semisynthetischen Opioids Buprenorphin sind im Stand der Technik mehrfach beschrieben. Hierbei wurden verschiedene Ansätze verfolgt, Buprenorphin in ausreichender Menge zur Verfügung zu stellen, insbesondere durch die Zugabe eines geeignet gewählten Permeationsförderers.

EP1174137 offenbart ein transdermales therapeutisches System enthaltend Buprenorphin und eine alpha-Hydroxysäure bzw. Milchsäure in Beispiel 6 mit 10 Gew.-%. EP 0 368 409 stellt beispielsweise Formulierungen zur transdermalen Darreichung von Buprenorphin-Salzen in einem Träger vor, umfassend a) ein polares Lösungsmittel ausgewählt aus der Gruppe der C3-C4 Diole, der C3-C6 Triole und Mischungen hiervon, sowie b) ein polares Lipidmaterial aus der Gruppe der Fettsäuren, Fettalkohole, Ester von Fettalkoholen, Ester von Fettsäuren und Mischungen hiervon, wobei das polare Lösungsmittel und das polare Lipidmaterial in einem Gewichtsverhältnis von ca. 60:40 bis ca. 99:1 vorhanden sind. Es wurde gefunden, dass Buprenorphin hiermit in wirksamen Mengen systemisch verabreicht werden kann.

WO 88/09676 offenbart gesättigte und ungesättigte Fettsäuren mit einer Kettenlänge von 8-18 C-Atomen oder C1-C4-Alkylester als Absorptionsförderer in wasserbasierten transdermalen therapeutischen Systemen. Buprenorphin ist hierin als über das TTS verabreichbarer Wirkstoff genannt. Lösungsansätze für nicht wasserbasierte Systeme werden nicht genannt.

WO 91/19474 beschreibt die transdermale Darreichung von Buprenorphin bei einer effektiven Abgaberate von 1 µg/h und einer Pflastergröße von 10 - 100 cm², entsprechend einer Abgaberate von ca. 0,05 - 5,0 µg/cm^{2*}h. Der Wirkstoff liegt hierbei gelöst in einem druckempfindlichen Haftkleber vor; ein Permeationsförderer kann vorhanden sein. Der Haftkleber kann Lösungsmittel sowohl organischer Natur als auch auf wässriger Basis beinhalten. Bevorzugte Permeationsförderer sind diverse Mischungen aus einem Ester und einem Ether.

Ein transdermales therapeutisches System basierend auf einem Polymermaterial und einer Kombination aus einem Weichmacher und einem Lösungsmittel wird in EP 0 430 019 dargestellt. Die Kombination aus einem Weichmacher und einem Lösungsmittel soll hierbei die Penetration des schlecht hautgängigen Buprenorphins verbessern.

WO 96/19975 beansprucht die transdermale Resorption von Buprenorphin mittels eines Hilfsstoffs, welcher unterkühlte Schmelzen bildet. Durch die Bildung von unterkühlten Schmelzen soll eine Rekristallisation des Wirkstoffs in der Polymermatrix verhindert werden. Charakteristisch für den Hilfsstoff ist hierbei, dass er bei üblicher Raumtemperatur fest ist. Als beispielhafte Hilfsstoffe werden Dodecanol oder Lävulinsäure genannt. Die Verwendung von Hilfsstoffen, welche üblicherweise bei Raumtemperatur fest sind, bringt erfahrungsgemäß häufig den Nachteil mit sich, dass die Klebeeigenschaften der Matrix unter dessen Verwendung verschlechtert werden, so dass für dessen Anwendung zusätzlich eine Fixierhilfe, meist in Form eines zusätzlichen Pflasters (Overtape) notwendig ist. Ein solches Pflaster ist bekannt unter dem Namen Transtec PRO® oder Norspan®. Dieser Aspekt wird umso wichtiger, je länger die Applikationsdauer des transdermalen Systems ist. Die Verwendung eines buprenorphinhaltigen transdermalen therapeutischen Systems gemäß WO 96/19975 über einen Applikationszeitraum von mindestens fünf Tagen ist beschrieben in WO 98/36728, wobei in diesem Dokument *in vivo* Blutspiegeldaten präsentiert werden.

Wünschenswert ist daher, ein transdermales therapeutisches System zur Verfügung zu haben, bei dem die Abgabe des Wirkstoffes Buprenorphin aus dem System über einen Applikationszeitraum von drei Tagen oder länger besser dosiert werden kann.

Die vorliegende Erfindung verfolgt hierzu im Vergleich zum Stand der Technik einen neuen Ansatz zur Lösung der oben genannten Probleme, nämlich die Abgabe des Buprenorphins aus dem transdermalen therapeutischen System durch Veränderung der Löslichkeit des Wirkstoffes in der Matrix zu beeinflussen.

Die Erfindung wird durch die Ansprüche definiert. Mit dem hier beschriebenen System ist es möglich, ein therapeutisches Pflaster mit dem Wirkstoff Buprenorphin über einen Applikationszeitraum von drei Tagen oder länger bereitzustellen, welches den Wirkstoff über den genannten Zeitraum kontinuierlich und in der zur Therapie erforderlichen Menge abgibt, dessen Polymermatrix den Wirkstoff in physikalisch stabiler Form beinhaltet und dessen Klebeeigenschaften so gestaltet sind, dass das TTS über einen Anwendungszeitraum von drei bis sieben Tagen gute Haftungseigenschaften aufweist.

Die vorliegende Erfindung betrifft daher ein buprenorphinhaltiges transdermales therapeutisches System, das umfasst:
(i) eine Rückschicht und
(ii) eine Polymermatrix, welche das Buprenorphin und zumindest eine α-Hydroxysäure und als weitere polymere Komponente Polyvinylpyrrolidon enthält.

Durch die Zugabe einer α-Hydroxysäure wird die Löslichkeit des Wirkstoffes in der Matrix des TTS stabilisiert, d.h. der Wirkstoff steht zu jedem Zeitpunkt der Applikationsdauer in ausreichender Menge in gelöster Form zur Verfügung. Diese durch die enthaltene α-Hydroxysäure frei zur Verfügung stehende Menge an Buprenorphin bestimmt nun das Abgabeverhalten aus dem Pflaster. Hierbei ist es möglich, durch die Zugabe der α-Hydroxysäure in geeignet gewählten Mengen den Konzentrationsgradienten des Buprenorphins an der Grenzfläche Pflaster/Haut einzustellen und dadurch die Menge des aus dem Pflaster abgegebenen Buprenorphins zu erhöhen und/oder das Abgabeverhalten des Buprenorphins über den Applikationszeitraum oder einen vorgegebenen Zeitraum während der Applikation zu regulieren.

Unter einer α-Hydroxysäure wird erfindungsgemäß eine Hydroxycarbonsäure verstanden, die zumindest eine Hydroxygruppe aufweist, die sich in α-Stellung zur Carboxygruppe befindet. Beispielhaft für eine α-Hydroxysäure können Milchsäure sowohl in Form der beiden Enantiomeren als auch in racemischer Form (DL-Milchsäure), α-Hydroxycaprylsäure, Weinsäure, Äpfelsäure, Gluconsäure, Mandelsäure, Glycolsäure, Zitronensäure sowie deren Gemische genannt werden.

Besonders vorteilhaft hat sich erwiesen, eine flüssige α-Hydroxysäure einzusetzen, wie die DL-Milchsäure und insbesondere die Milchsäure Ph.Eur., welche von der Firma Sigma Aldrich, VWR oder Carl Roth erhältlich ist. Diese Verbindungen, die bei Raumtemperatur (25 °C) flüssig sind, beeinflussen nicht nur die Abgabeeigenschaften des Buprenorphins in der gewünschten Weise, sondern können aufgrund ihrer flüssigen Konsistenz auch die Klebeeigenschaften der Polymermatrix verbessern, sodass das TTS ohne die ergänzende Zugabe weiterer Hilfsstoffe und insbesondere von Weichmachern bessere Trageeigenschaften aufweist.

Durch die Zugabe der α-Hydroxysäure in eine vorgegebene Polymermatrix kann die Sättigungslöslichkeit des Buprenorphins in der Polymermatrix im Vergleich mit dieser, die α-Hydroxysäure nicht enthaltenden Matrix erhöht werden. Eine vorteilhafte Menge der α-Hydroxysäure ist hierbei 1 bis 10 Gew.-%, bezogen auf das Gewicht der wirkstoffhaltigen Polymermatrix. Durch die mögliche Erhöhung der Sättigungslöslichkeit ist es mit dem hier beschriebenen TTS möglich, im Vergleich mit Systemen ohne den Zusatz von α-Hydroxysäure mehr Wirkstoff in eine vorgegebene Matrix einzubringen. Darüber hinaus kann aufgrund der höheren Sättigungslöslichkeit die physikalische Stabilität, d.h. die Vermeidung der Rekristallisation des Wirkstoffs in der Matrix, und somit die Lagerstabilität des Systems verbessert werden.

Soweit in der vorliegenden Erfindung auf die Sättigungslöslichkeit bzw. die Sättigungskonzentration von Buprenorphin Bezug genommen wird, beziehen sich diese Angaben auf eine Temperatur von 25°C. Die Sättigungslöslichkeit wird wie folgt bestimmt: es werden eine Reihe von Pflastern mit steigenden Buprenorphinkonzentrationen hergestellt und bei 25°C gelagert. Die Pflaster werden über einen Zeitraum von sechs Monaten beobachtet. Die höchste Konzentration, bei der nach sechs Monaten noch kein Wirkstoff auskristallisiert ist (bzw. als Feststoff ausgefallen ist), stellt die untere Grenze der Sättigungslöslichkeit dar. Die niedrigste Konzentration, bei der nach sechs Monaten Wirkstoff auskristallisiert ist (bzw. als Feststoff ausgefallen ist), stellt die obere Grenze der Sättigungslöslichkeit dar. Die Sättigungslöslichkeit liegt dann innerhalb des durch die beiden Grenzwerte gebildeten Bereichs. Die Genauigkeit der Messmethode kann erhöht werden, indem die Abstände zwischen den Konzentrationen verringert werden. Die Beobachtung erfolgt optisch (mit dem Auge).

Durch den Zusatz der α-Hydroxysäure wird die physikalische Stabilität des Wirkstoffs in der Polymermatrix vorteilhaft beeinflusst. Das heißt, dass eine Rekristallisation des Buprenorphins in der Polymermatrix durch den Zusatz von α-Hydroxysäure verhindert oder zumindest deutlich verzögert wird. Der Gehalt an α-Hydroxysäure zur Verbesserung der physikalischen Stabilität bzw. Vorbeugung der Rekristallisation des Buprenorphins liegt vorzugsweise im Bereich von 1 bis 10 Gew.%, bevorzugt im Bereich von 2 bis 8 Gew.% und insbesondere bevorzugt im Bereich von 3 bis 7 Gew.%.

In Bezug auf den im System enthaltenen Wirkstoff Buprenorphin sind unter diesem Begriff in dieser Beschreibung sowohl die freie Base als auch ein physiologisch unbedenkliches Salz, ein Hydrat und/oder ein Solvat von Buprenorphin zu verstehen.

Wie weiter oben ausgeführt wurde, ist es durch den Zusatz der α-Hydroxysäure in eine vorgegebene Polymermatrix möglich, die Löslichkeit zu beeinflussen, wodurch im Vergleich mit dem System ohne α-Hydroxysäure die Beladung der Matrix mit dem Wirkstoff deutlich erhöht werden kann. Daher kann der Wirkstoff Buprenorphin in der Polymermatrix in einer Konzentration von bis zu 30 Gew.-%, bevorzugt im Bereich von 5 bis 20 Gew.-%, vorzugsweise im Bereich von 8 bis 15 Gew.-% und insbesondere bevorzugt im Bereich von 9 bis 13 Gew.-% enthalten sein. Die Einheit Gewichtsprozent bezieht sich hierbei auf das Gesamtgewicht der Polymerschicht einschließlich des Wirkstoffs und weiterer Bestandteile. Das Massenverhältnis von Wirkstoff zu α-Hydroxysäure kann in einer Ausführungsform im Bereich von 10:1 bis 1:1, bevorzugt 6:1 bis 1,5:1 und insbesondere im Bereich von 3:1 bis 2:1 liegen. Der Buprenorphingehalt des TTS liegt gewöhnlich im Bereich von 0,05 bis 2 mg/cm², bevorzugt im Bereich von 0,2 bis 1,5 mg/cm², insbesondere im Bereich von 0,5 bis 1,2 mg/cm².

Die Fläche des wirkstoffhaltigen TTS liegt üblicherweise im Bereich von 2,5 bis 100 cm², bevorzugt im Bereich von 4 bis 60 cm², insbesondere im Bereich von 5 bis 50 cm², wobei die buprenorphinhaltige Polymermatrix nach einer Ausführungsform ein Flächengewicht im Bereich von 30 bis 120 g/m², bevorzugt 40 bis 100 g/m², insbesondere im Bereich von 50 bis 90 g/m² aufweist.

Nach einer Ausführungsform besteht das TTS aus einer wirkstoffhaltigen Polymerschicht, jedoch kann das erfindungsgemäße System auch zusätzliche wirkstoffhaltige Schichten mit gleicher oder unterschiedlicher Wirkstoffbeladung oder auch wirkstofffreie Schichten enthalten.

Für die Herstellung der Polymermatrix eignen sich grundsätzlich alle Polymere, welche für die transdermalen Systeme herkömmlich eingesetzt werden und physiologisch unbedenklich sind. Beispiele hierfür sind etwa Polyacrylate, Polymethacrylate, Polystyrole, Polybutadiene, Polyisoprene, Polyisobutylene und Polysilicone sowie deren Gemische.

Im Hinblick auf ihre vorteilhafte Stabilität und ihre guten Klebeeigenschaften sowie die Möglichkeit, organische Lösemittel in der Matrix einsetzen zu können, werden nach einer Ausführungsform die Homopolymere und Copolymere der Acrylsäure, Methacrylsäure, Acrylsäureester oder Methacrylsäureester eingesetzt. Besonders vorteilhaft sind die Polymere aus der Gruppe der Polyacrylate, insbesondere die Copolymere der Acrylsäurealkylester und/oder Methacrylsäurealkylester, deren Alkylgruppe 1 bis 12 Kohlenstoffatome besitzt, mit einem Comonomer mit sauren funktionellen Gruppen. Diese Copolymere umfassen hierbei polymere Verbindungen aus Acrylsäureestern, wie zum Beispiel Methylacrylat, Ethylacrylat, 2-Ethylhexylacrylat, Hydroxyethylacrylat, Butylacrylat, Butylmethylacrylat und Isooctylacrylat und bestehen für gewöhnlich zu 50 bis 95 Gew.-% aus dem Hauptmonomer bzw. den Hauptmonomeren, zu 2 bis 40 Gew.- % aus einem modifizierenden Monomer oder Monomerengemisch und zu 2 bis 20 Gew.-% aus einem oder mehreren Monomeren mit funktionellen Gruppen.

Unter einer sauren funktionellen Gruppe ist in diesem Zusammenhang eine Carboxygruppe (-COOH) zu verstehen. Ihre Funktion in einem haftklebenden Polymer ist die der Feuchtigkeitsaufnahme; darüber hinaus fungiert sie auch als Bindungsstelle zur intermolekularen Vernetzung.

Dem Fachmann sind Polyacrylate mit funktionellen Carboxygruppen bekannt, die zum Beispiel von der Firma Henkel unter dem Produktnamen DURO-TAK® 387-2051, DURO-TAK® 387-2052, DURO-TAK® 387-2054, DURO-TAK® 87-2074, DURO-TAK® 387-2353, DURO-TAK® 87-2852, DURO-TAK® 87-2677, DURO-TAK® 87-2194 oder DURO-TAK® 87-2196 kommerziell erhältlich sind.

Zur Modifizierung bzw. Verbesserung der Fließ- und/oder Scherkräfte des Polyacrylats kann dem erfindungsgemäßen TTS ein Vernetzer zugegeben werden. Die Vernetzung von Acrylat-Haftklebern erfolgt für gewöhnlich über die in das Polymer eingebrachten funktionellen Gruppen des Comonomers, im Falle der bevorzugten sauren Polyacrylatcopolymere über die Carboxygruppen, und ist im Stand der Technik bekannt. Sehr häufig werden als Vernetzer Komplexbildner zugesetzt, welche mit den funktionellen Gruppen des Polymers wechselwirken, wie zum Beispiel Zink- und Titaniumverbindungen. Die Konzentration an Vernetzer übersteigt dabei selten 1 Gew.-%.

Unter guten Klebeeigenschaften ist zu verstehen, dass die Polymermatrix bei der Applikation auf der Haut haftet. Dies schließt nicht aus, dass zur Verbesserung der Haftung des transdermalen therapeutischen Systems zusätzliche Substanzen Verwendung finden und/oder ein das wirkstoffhaltige System überlappendes Overtape vorgesehen wird.

Nach einer Ausführungsform ist die Klebkraft des buprenorphinhaltigen TTS derart, dass das System über einen Applikationszeitraum von mindestens drei, bevorzugt vier bis fünf, insbesondere bevorzugt sechs bis sieben Tage sicher auf der Haut hält und ohne oder nur geringfügigen Hautschädigungen oder Irritationen von der Haut entfernt werden kann. Insofern im Rahmen der vorliegenden Erfindung auf die Klebkraft Bezug genommen wird, erfolgt deren Messung nach der entsprechenden DIN-Vorschrift wie z. B. DIN EN1939.

Die Polymermatrix kann selbstverständlich weitere Zusatzstoffe enthalten, wie sie gewöhnlich in transdermalen Systemen eingesetzt werden. Beispiele hierfür sind die Permeation fördernde Substanzen, Weichmacher, Klebrigmacher, Lösungsmittel sowie Kristallisationsinhibitoren und weitere polymere Substanzen.

Zur Verbesserung der chemischen Stabiliät des Buprenorphins im transdermalen System können zusätzlich Antioxidantien zugegeben werden. Bevorzugte Antioxidantien sind hierbei Butylhydroxytoluol, Butylhydroxyanisol, Vitamin C-Palmitat, Tocopherol und dessen Derivate.

Geeignete Permeationsförderer für den Wirkstoff sind im Stand der Technik bekannt. Diese können ausgewählt sein aus der Gruppe der Fettsäuren, Fettalkohole, Ester von Fettalkoholen, Ester von Fettsäuren und Mischungen hiervon. Nach einer Ausführungsform können dem transdermalen therapeutischen System Fettsäureester, insbesondere Ester der Ölsäure und Ester von Sorbitan (1,4-Sorbitanhydrid) und mittel- und langkettiger Fettsäuren zugegeben werden. Diese Permeationsförderer können vorteilhaft auch als Weichmacher agieren und/oder auch die Abgabe des Wirkstoffs aus dem transdermalen System modulieren. Ein Beispiel für einen Fettsäureester der Ölsäure ist Oleyloleat (syn. Ölsäureoleylester, Decyloleat, Octadecensäuredecylester), als Beispiele für Ester von Sorbitan können Sorbitanmonolaurat (syn. Sorbitanmonododecanoat), Sorbitanmonostearat, Sorbitantristearat, Sorbitanmonooleat und Sorbitanmonopalmitat sowie deren Gemische genannt werden. Oleyloleat ist kommerziell zum Beispiel unter der Bezeichnung Cetiol®, Sorbitanmonolaurat unter der Bezeichnung Span 20 verfügbar. Die Konzentration des Permeationsförderers beträgt üblicherweise bis 25 Gew.-%, bevorzugt 3 bis 22 Gew.-%, insbesondere 5 bis 20 Gew.-%.

Als weitere polymere Verbindung ist Polyvinylpyrollidon (PVP) in der Polymermatrix enthalten.

Die Rückschicht des TTS befindet sich bei der Applikation des Systems auf der der menschlichen Haut abgewandten Seite der wirkstoffhaltigen Polymermatrix. Die Rückschicht kann hierbei okklusive oder nicht okklusive Eigenschaften aufweisen. Derartige Rückschichten können in bevorzugter Ausführungsform aus Polyolefinen, insbesondere Polyethylen, oder aus Polyestern sowie Polyurethanen bestehen. Auch Schichten, die mehrere verschiedene Polymere übereinander angeordnet beinhalten, sind vorteilhafterweise einsetzbar. Ein besonders bevorzugtes Material für die Rückschicht ist ein Polyethylenterephthalat, das von der Firma Mitsubishi unter der Bezeichnung Hostaphan vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymer, Papier, das gegebenenfalls beschichtet sein kann, textile Gewebe, Polyesterfolien, wie Polyethylentherephthalatfolien. Besonders bevorzugt sind Aluminiumfolie und Polymer-Metall-Compositmaterialien. Die Dicke der Rückschicht ist, wie im Stand der Technik üblich, z.B. 5 µm bis 300 µm, insbesondere 10 bis 150 µm nominelle Dicke.

Weitere Merkmale der Erfindung ergeben sich aus der nachfolgenden Beschreibung von Ausführungsbeispielen in Verbindung mit den Ansprüchen sowie den beiliegenden Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können bei erfindungsgemäßen Ausführungsformen die bei den nachstehend erläuterten Ausführungsbeispielen angeführten Merkmale in von den Beispielen abweichender Anzahl und Kombination verwirklicht sein. Bei der nachfolgenden Erläuterung einiger Ausführungsbeispiele der Erfindung wird auf die beiliegenden Figuren Bezug genommen, von denen
Fig. 1a einen Querschnitt durch ein transdermales therapeutisches System in einer schematischen Darstellung zeigt,
Fig. 1b einen Querschnitt durch ein transdermales therapeutisches System mit einem Overtape in einer schematischen Darstellung zeigt, und
Fig. 2 die Wirkstofffreisetzung verschiedener TTS in Abhängigkeit von der Zeit darstellt.

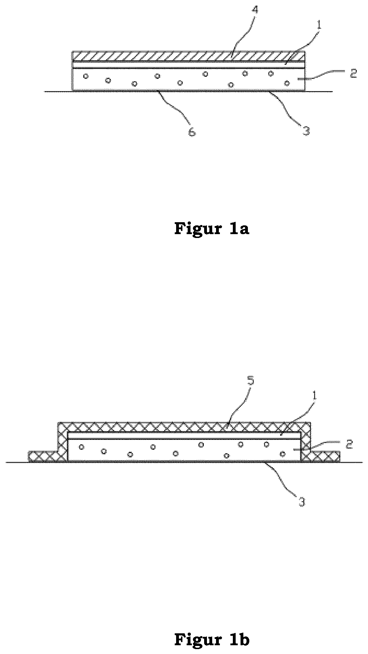

Der Aufbau eines transdermalen therapeutischen Systems im Querschnitt ist in Figur 1a und Figur 1b dargestellt. Das transdermale therapeutische System besteht aus einer Rückschicht 1, einer wirkstoffhaltigen Polymermatrix 2 und einer Schutzfolie (Abziehfolie) 3, welche dem Schutz der wirkstoffhaltigen Matrix dient und vor der Applikation des Pflasters entfernt wird. Natürlich kann das TTS auch mehrere unterschiedliche wirkstoffhaltige oder wirkstofffreie Polymermatrices enthalten. Die Rückschicht 1 des TTS besitzt die gleiche Fläche wie die wirkstoffhaltige Polymermatrix 2, so dass weder die Rückschicht über die wirkstoffhaltige Matrix noch die wirkstoffhaltige Matrix über die Rückschicht hinausragt. Die Schutzfolie 3 ragt, wie es für TTS üblich ist, über die wirkstoffhaltige Polymermatrix bzw. die Rückschicht hinaus.

Zur Erleichterung der Entnahme aus der Pflasterverpackung ist eine Abdeckschicht 4 (Figur 1a) vorhanden, die im Wesentlichen lose auf der Rückschicht aufliegt und beispielsweise durch elektrostatische Kräfte auf dieser haftet.

Nach einer Ausführungsform kann das TTS auch von einer Fixierhilfe (Overtape) 5 (Figur 1b) überdeckt sein, welche fest auf der Rückschicht aufgebracht ist und die wirkstoffhaltige Matrix überragt. Dieses Overtape besteht üblicherweise aus einer Rückschicht und einer im Allgemeinen wirkstofffreien haftklebenden Polymermatrix und dient zur Fixierung des buprenorphinhaltigen TTS auf der Haut. Fixierhilfe bzw. Overtapes sind im Stand der Technik bekannt. Zur Verbesserung der Entnahme aus der Pflasterverpackung kann auch hier eine Abdeckschicht 4 vorhanden sein, welche lose auf dem Overtape aufliegt.

Die Rückschicht des Overtapes befindet sich bei Applikation des Systems auf der der menschlichen Haut abgewandten Seite des TTS. Die Ausführungsformen umfassen sowohl okklusive als auch nicht okklusive Rückschichten. Derartige Rückschichten können in bevorzugter Ausführungsform aus Polyolefinen, insbesondere Polyethylen, oder aus Polyestern sowie Polyurethanen bestehen. Auch Schichten, die mehrere verschiedene Polymere übereinander angeordnet beinhalten, sind einsetzbar. Ein besonders bevorzugtes Material für die Rückschicht ist ein Polyolefin, das von der Firma Mylan Technologies Inc. unter der Bezeichnung Mediflex®1000 vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymer, Papier, das gegebenenfalls beschichtet sein kann, textile Gewebe, Polyesterfolien, wie Polyethylentherephthalatfolien. Besonders bevorzugt sind Polyolefine und Polymer-Metall-Compositmaterialien. Die Dicke der Rückschicht ist, wie im Stand der Technik üblich, z.B. 10 µm bis 1000 µm, insbesondere 40 bis 500 µm nominelle Dicke.

Die zum Schutz der wirkstoffhaltigen Matrix und vor Applikation zu entfernende Schutzfolie 3 kann mit einem Schnitt 6 (Figur 1a) versehen sein, welche das Abziehen der Schutzfolie von der wirkstoffhaltigen Matrix erleichtert und eine Pflasterapplikation ohne Berührung der wirkstoffhaltigen Matrix mit den Händen einfach ermöglicht.

Die Schutzfolie ist bevorzugt aus polymerem Material hergestellt, das gegebenenfalls auch metallisiert sein kann. Beispiele für bevorzugt eingesetzte polymere Materialien sind Polyurethane, Polyvinylacetat, Polyvinylidenchlorid, Polypropylen, Polycarbonat, Polystyrol, Polyethylen, Poylethylenterephthalat, Polybutylenterephthalat sowie gegebenenfalls mit entsprechenden Polymeren oberflächenbeschichtetes Papier. Bevorzugt handelt es sich hierbei um eine einseitig oder beidseitig fluorpolymerbeschichtete oder siliconisierte Schutzfolie. Besonders bevorzugt sind handelsübliche fluorpolymerbeschichtete oder siliconisierte Polyesterfolien, wie die einseitig siliconisierten Handelsprodukte Primeliner 100 µm (Firma Loparex) oder die einseitig fluorpolymerbeschichteten Handelsprodukte Scotchpak 1022 oder Scotchpak 9742 (Firma 3M).

Die Herstellung eines transdermalen therapeutischen Systems erfolgt beispielsweise, indem man zunächst den Wirkstoff in einem hierfür geeigneten Lösungsmittel löst, diese Lösung im Anschluss zur Polymer-/Hilfsstoffmischung zugibt und bis zur Homogenität mischt. Die wirkstoffhaltige Beschichtungslösung wird dann als gleichmäßige Schicht auf der Rückschicht aufgebracht, getrocknet und mit der Schutzfolie zu einem Laminat kaschiert. In einem nächsten Schritt werden aus dem Laminat Pflaster gewünschter Form und Größe ausgestanzt, optional mit einem Overtape versehen und wahlweise mit einer siliconisierten oder fluorpolymerbeschichteten Abdeckschicht.

In einer Ausführungsform erzielt das buprenorphinhaltige TTS die gleiche oder zumindest eine möglichst ähnliche Blutplasmaspiegelkurve wie die bekannten Marktprodukte Norspan®, Transtec® Pro oder Buprenorphin AWD Matrix®. Insbesondere sind die TTS zu dem Produkt Norspan® bioäquivalent.

Die buprenorphinhaltigen TTS können zur Schmerztherapie eingesetzt werden. Ein weiteres Indikationsgebiet umfasst die Therapie der Drogenabhängigkeit.

### Beispiel 1 - Physikalische Stabilität des TTS

Die physikalische Stabilität des Buprenorphins in einer Milchsäure enthaltenden Polymermatrix wurde über einen Testzeitraum von 2 Monaten bei 25 und 40°C untersucht. Zum Vergleich wurde auch ein entsprechendes TTS ohne Zusatz von Milchsäure untersucht.

Hierfür wurde Buprenorphin in Tetrahydrofuran unter Rühren vollständig gelöst. Oleyloleat wird zusammen mit Milchsäure in die Acrylat-Vinylacetatcopolymer-Lösung (zum Beispiel DURO TAK® 387-2052, DURO TAK® 387-2054) gegeben und gerührt. Anschließend wird Polyvinylpyrollidon (PVP, zum Beispiel Povidon K90) unter Rühren auf die Beschichtungsmasse gestreut und so lange gerührt, bis eine klumpenfreie, klare Masse entsteht. Eine vorteilhafte Menge an Polyvinylpyrrolidon trägt in diesem Schritt dazu bei, die Viskosität der Beschichtungslösung im Hinblick auf eine gute Beschichtbarkeit der Masse optimal einzustellen. Im Anschluss wird die Wirkstofflösung zugegeben und bis zur vollständigen Homogenität gerührt.

Die homogene Beschichtungsmasse wird als dünner Film auf einer fluoropolymerbeschichteten Folie (zum Beispiel Scotchpak™ 1022) aufgetragen. Die Lösemittel werden im Trockenofen bei einer Temperatur von 60 bis 120 °C abgedampft und der getrocknete Matrixfilm wird mit einer Rückfolie aus Polyethylenterephthalat (zum Beispiel Hostaphan MN 15 MED oder Hostaphan MN 12 MED) kaschiert. Aus dem Laminat werden Pflaster gestanzt. Optional werden die Pflaster mit einem Overtape versehen.

Eine Übersicht der Zusammensetzung des erfindungsgemäßen Beispiels 1 sowie des Vergleichsbeispiels ist in Tabelle 1 dargestellt.

**Tabelle 1**

| Beispiel | Rezeptur | Aussehen bei 25°C (M ikroskop) | Aussehen bei 40°C (Mikroskop) |
|---|---|---|---|
| | | Kristalle | Kristalle |
| Beispiel 1 | 10% Buprenorphin, 10% PVP, 10% Oleyl-oleat, 10% Milchsäure, Polyacrylat DURO-TAK DT 2052 | nicht vorhanden | nicht vorhanden |
| Vergleichs-beispiel | 10% Buprenorphin, 10% PVP, 10% Oleyloleat, Polyacrylat DURO-TAK DT 2052 | nicht vorhanden | vorhanden |

Wie aus Tabelle 1 ersichtlich, ist nach einem Testzeitraum von 2 Monaten bei 25 °C und 40 °C bei Verwendung von Milchsäure keine Kristallisation des Wirkstoffs zu beobachten; das Buprenorphin wird durch den Zusatz von Milchsäure in der Polymermatrix physikalisch stabilisiert und somit kann einer Rekristallisation des Wirkstoffs vorgebeugt werden.

### Beispiel 2 - in vitro Hautpermeation

Der Einfluss von Milchsäure auf die Wirkstoffpermeation wurde in diesem Beispiel untersucht. Es werden 2 buprenorphinhaltige TTS A und B wie in Beispiel 1 beschrieben hergestellt, wobei die enthaltene Milchsäuremenge variiert wurde.

Die Zusammensetzungen dieser TTS sind in Tabelle 2 dargestellt.

**Tabelle 2**

| | TTS A(%) | TTSB (%) |
|---|---|---|
| Buprenorphin | 11 | 11 |
| PVP | 10 | 10 |
| Milchsäure | 3 | 6 |
| Oleyloleat | 15 | 15 |
| DuroTak 2052/2054 | 61 | 58 |

Als Vergleichsbeispiel dient das kommerziell erhältliche buprenorphinhaltige TTS Norspan®.

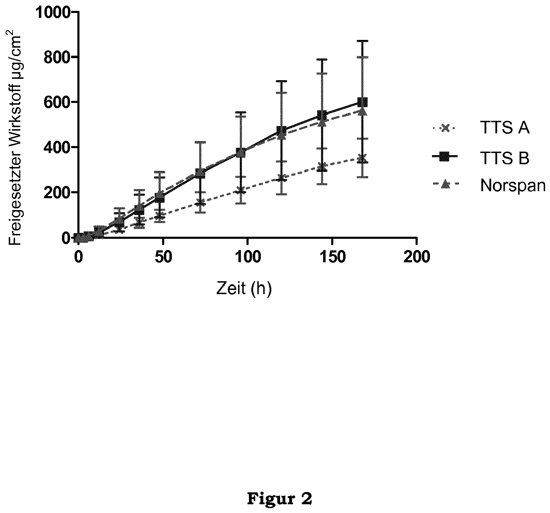

Die *in vitro* Hautpermeation der TTS A und B sowie des Vergleichsbeispiels Norspan® durch Schweineohrhaut wurde mit Hilfe von statischen Diffusionszellen bei einer Temperatur von 32 °C bestimmt. Die vergleichenden Ergebnisse sind in der Figur 2 dargestellt: Die TTS A und B zeigen während der Testzeit von 168 Stunden eine kontinuierliche und sehr vergleichbare *in vitro* Permeation des Buprenorphins zu dem kommerziell erhältlichen Produkt Norspan®.

## Patentansprüche

1. Buprenorphinhaltiges transdermales therapeutisches System (TTS) umfassend (i) eine Rückschicht und (ii) eine Polymermatrix, die Buprenorphin, zumindest eine α-Hydroxysäure und als weitere polymere Komponente Polyvinylpyrrolidon enthält.

2. TTS nach Anspruch 1, wobei die α-Hydroxysäure bei Raumtemperatur flüssig ist.

3. TTS nach Anspruch 1 oder 2, wobei es sich bei der α-Hydroxysäure um Milchsäure handelt.

4. TTS nach einem der vorhergehenden Ansprüche, wobei die α-Hydroxysäure in einer Konzentration im Bereich von 1 bis 10 Gew.-%, vorzugsweise im Bereich von 2 bis 8 Gew.-% und insbesondere bevorzugt im Bereich von 3 bis 7 Gew.-% vorliegt.

5. TTS nach einem der vorhergehenden Ansprüche, wobei das Buprenorphin in Form der Base, eines Salzes, als Hydrat und/oder Solvat vorliegt.

6. TTS nach einem der vorhergehenden Ansprüche, wobei das Buprenorphin in einer Konzentration im Bereich von 5 bis 20 Gew.-%, bevorzugt im Bereich von 8 bis 15 Gew.-% und insbesondere bevorzugt im Bereich von 9 bis 13 Gew.-% in der Polymermatrix vorliegt.

7. TTS nach einem der vorhergehenden Ansprüche, wobei der Gehalt an Buprenorphin im TTS im Bereich von 0,05 bis 2 mg/cm2, bevorzugt im Bereich von 0,2 bis 1,5 mg/cm2 und insbesondere im Bereich von 0,5 bis 1,2 mg/cm2 liegt.

8. TTS nach einem der vorhergehenden Ansprüche, wobei das Polymer der Polymermatrix (ii) ein Acrylatcopolymer ist.

9. TTS nach Anspruch 8, wobei das Acrylatcopolymer funktionelle Carboxygruppen enthält.

10. TTS nach Anspruch 8 oder 9, wobei das Acrylatcopolymer einen Vernetzer enthält.

11. TTS nach einem der vorhergehenden Ansprüche, wobei der Polymermatrix ein oder mehrere Sorbitanfettsäureester zugesetzt sind, insbesondere Sorbitanmonolaurat.

12. TTS nach einem der vorhergehenden Ansprüche, wobei der Polymermatrix ein oder mehrere Ester der Olsäure, insbesondere Oleyloleat, zugesetzt sind.

13. TTS nach einem der vorhergehenden Ansprüche, wobei das Buprenorphin über einen Zeitraum von mindestens 3 Tagen in einer therapeutisch wirksamen Dosis abgegeben wird.

14. TTS nach einem der vorhergehenden Ansprüche zur Verwendung für die Schmerztherapie.

15. TTS nach einem der Ansprüche 1 bis 13 zur Verwendung für die Therapie der Drogenabhängigkeit.

## Claims

1. A buprenorphine-containing transdermal therapeutic system (TTS) comprising (i) a backing layer and (ii) a polymer matrix containing buprenorphine, at least one α-hydroxy acid and, as a further polymeric component, polyvinylpyrrolidone.

2. TTS according to claim 1, wherein the α-hydroxy acid is liquid at room temperature.

3. TTS according to claim 1 or 2, wherein the α-hydroxy acid is lactic acid.

4. TTS according to any one of the preceding claims, wherein the α-hydroxy acid is present in a concentration in the range of 1% by weight to 10% by weight, preferably in the range of 2% by weight to 8% by weight, and in particular preferably in the range of 3% by weight to 7% by weight.

5. TTS according to any one of the preceding claims, wherein the buprenorphine is present in the form of the base, a salt, as hydrate and/or solvate.

6. TTS according to any one of the preceding claims, wherein the buprenorphine is present in the polymer matrix in a concentration in the range of 5% by weight to 20% by weight, preferably in the range of 8% by weight to 15% by weight, and in particular preferably in the range of 9% by weight to 13% by weight.

7. TTS according to any one of the preceding claims, wherein the content of buprenorphine in the TTS is in the range from 0.05 mg/cm² to 2 mg/cm², preferably in the range from 0.2 mg/cm² to 1.5 mg/cm², and in particular in the range from 0.5 mg/cm² to 1.2 mg/cm².

8. TTS according to any one of the preceding claims, wherein the polymer of the polymer matrix (ii) is an acrylate copolymer.

9. TTS according to claim 8, wherein the acrylate copolymer contains carboxy functional groups.

10. TTS according to claim 8 or 9, wherein the acrylate copolymer contains a crosslinker.

11. TTS according to any one of the preceding claims, wherein one or more sorbitan fatty acid esters, in particular sorbitan monolaurate, are added to the polymer matrix.

12. TTS according to any one of the preceding claims, wherein one or more esters of oleic acid, in particular oleyl oleate, are added to the polymer matrix.

13. TTS according to any one of the preceding claims, wherein the buprenorphine is delivered over a period of at least 3 days in a therapeutically effective dose.

14. TTS according to any one of the preceding claims for use in pain therapy.

15. TTS according to any one of claims 1 to 13 for use in the therapy of drug addiction.

## Revendications

1. Système thérapeutique transdermique (STT) contenant de la buprénorphine, le STT comprenant (i) une couche arrière et (ii) une matrice polymère contenant la buprénorphine, au moins un acide alpha hydroxylé et comme autre composante polymère de la polyvinylpyrrolidone.

2. STT selon la revendication 1, où l'acide alpha hydroxylé est liquide à température ambiante.

3. STT selon la revendication 1 ou 2, où l'acide alpha hydroxylé est de l'acide lactique.

4. STT selon l'une des revendications précédentes, où l'acide alpha hydroxylé est présent en une concentration dans la plage allant de 1 à 10% en poids, de préférence dans la plage allant de 2 à 8% en poids, très préférentiellement dans la plage allant de 3 à 7% en poids.

5. STT selon l'une des revendications précédentes, où la buprénorphine est présente sous forme de base, sous forme d'un sel, sous forme hydratée et/ou sous forme solvatée.

6. STT selon l'une des revendications précédentes, où la buprénorphine est présente dans la matrice polymère en une concentration dans une plage allant de 5 à 20% en poids, de préférence dans une plage allant de 8 à 15% en poids, très preférentiellement dans une plage allant de 9 à 13% en poids.

7. STT selon l'une des revendications précédentes, où la teneur en buprénorphine dans le STT est dans la plage allant de 0,05 à 2 mg/cm², de préférence dans la plage allant de 0,2 à 1,5mg/cm², très préférentiellement dans la plage allant de 0,5 à 1,2 mg/cm².

8. STT selon l'une des revendications précédentes, où le polymère de la matrice polymère (ii) est un copolymère d'acrylate.

9. STT selon la revendication 8, où le copolymère d'acrylate contient des groupements carboxyle fonctionnels.

10. STT selon la revendication 8 ou 9, où le copolymère d'acrylate contient un agent de réticulation.

11. STT selon l'une des revendications précédentes, où un ou plusieurs esters d'acide gras de sorbitane, en particulier du monolaurate de sorbitane, sont ajoutés à la matrice polymère.

12. STT selon l'une des revendications précédentes, où un ou plusieurs esters d'acide oléique, en particulier de l'oléate d'oléyle, sont ajoutés à la matrice polymère.

13. STT selon l'une des revendications précédentes, où la buprénorphine est administrée en une dose thérapeutiquement efficace sur une période d'au moins 3 jours.

14. STT selon l'une des revendications précédentes pour l'utilisation dans la thérapie de la douleur.

15. STT selon l'une des revendications 1 à 13 pour l'utilisation dans la thérapie de la dépendance aux drogues
